# EUROPEAN PATENT APPLICATION

(11) **EP 4 520 744 A1**
(43) Date of publication of application: **12.03.2025**
(21) Application number: 23799521.2
(22) Date of filing: 08.05.2023
(51) Int. Cl.: C05F 11/08, C12N 1/20

(54) **FERTILIZER FOR AGRICULTURAL PRODUCTS**

(30) Priority: 06.05.2022 JP 2022076662
(71) Applicant: Symbiobe Inc., Kyoto-shi, Kyoto 615-8245 (JP); Kyoto University, Kyoto-shi, Kyoto 606-8501 (JP)
(72) Inventor: NUMATA, Keiji, Kyoto-shi, Kyoto 606-8501 (JP); YAGI, Shamitha Rao, Kyoto-shi, Kyoto 606-8501 (JP); NAKAZAKI, Tetsuya, Kyoto-shi, Kyoto 606-8501 (JP); NAKANO, Ryohei, Kyoto-shi, Kyoto 606-8501 (JP); MOTOKI, Ko, Kyoto-shi, Kyoto 606-8501 (JP); IWAHASHI, Yu, Kyoto-shi, Kyoto 606-8501 (JP); KOBAYASHI, Masaru, Kyoto-shi, Kyoto 606-8501 (JP); OCHIAI, Kumiko, Kyoto-shi, Kyoto 606-8501 (JP)
(74) Representative: D Young & Co LLP
(86) International application number: PCT/JP2023/017257
(87) International publication number: WO 2023/214592

(57) **Abstract**

The present invention provides an agricultural fertilizer having a high nitrogen content. The present invention provides an agricultural fertilizer containing a crushed product of a marine purple photosynthetic bacterium and having a nitrogen content of 8.0% by mass or more.

## Description

### Technical Field

The present invention relates to an agricultural fertilizer.

### Background Art

Most fertilizers for agricultural crops are produced by using fossil resources as raw materials. For example, most nitrogenous fertilizers are chemically synthesized through the Haber-Bosch process. A method for producing a fertilizer by using a photosynthetic bacterium has been developed as a method for producing a fertilizer without relying on fossil resources.

For example, Patent Literature 1 discloses an agricultural/horticultural fertilizer containing an extract from a cyanobacterium or an extract from a photosynthetic bacterium belonging to the genus *Halobacterium,* the genus *Rhodopseudomonas,* or the genus *Rhodospirillum.* In particular, Patent Literature 1 discloses that an extract from *Rhodopseudomonas blastica* can be used as an extract from a photosynthetic bacterium for an agricultural/horticultural fertilizer.

### Citation List

### Patent Literature

Patent Literature 1: Japanese Patent Laid-Open No. 11-335191

### Summary of Invention

### Technical Problem

However, Patent Literature 1 does not regard giving a high nitrogen content to the agricultural/horticultural fertilizer as an objective, and it is assumed that no agricultural fertilizer having a high nitrogen content has been provided yet.

An objective of the present invention is to provide an agricultural fertilizer having a nitrogen content as high as 8.0% by mass or more.

### Solution to Problem

To achieve the aforementioned objective, the present inventors have diligently endeavored to find that use of a marine purple photosynthetic bacterium enables production of an agricultural fertilizer having a high nitrogen content, thus completing the present invention.

Specifically, the present invention is as follows.
[1] An agricultural fertilizer containing a crushed product of a marine purple photosynthetic bacterium and having a nitrogen content of 8.0% by mass or more.
[2] The agricultural fertilizer according to [1], wherein the ratio of the carbon content to the nitrogen content (C/N) is 3.0 or more and 7.0 or less.
[3] The agricultural fertilizer according to [1] or [2], wherein the agricultural fertilizer is a solid fertilizer.
[4] The agricultural fertilizer according to any one of [1] to [3], wherein the agricultural fertilizer has slow-release properties and thereby continuously acts for 30 days or more.

### Advantageous Effect of Invention

The present invention can provide an agricultural fertilizer having a nitrogen content as high as 8.0% by mass or more.

### Brief Description of Drawings

[Figure 1] Figure 1 shows results of cultivation of Japanese mustard spinach plants in Example 1.
[Figure 2] Figure 2 shows the temporal variations of broccoli plants in cultivation thereof in Example 4.
[Figure 3] Figure 3 shows results of harvesting of broccoli plants in Example 4.
[Figure 4] Figure 4 shows the temporal variations of the SPAD values of broccoli plants in Example 4. *** in the figure indicates significant difference in testing by one-way analysis of variance at a significance level of 0.001.
[Figure 5] Figure 5 shows the temporal variations of the maximum leaf lengths of broccoli plants in Example 4. *, **, and *** in the figure indicate significant difference in testing by one-way analysis of variance at a significance level of 0.05, of 0.01, and of 0.001, respectively.
[Figure 6] Figure 6 shows the temporal variations of the maximum flower bud diameter of broccoli plants in Example 4. ** and *** in the figure indicate significant difference in testing by one-way analysis of variance at a significance level of 0.01 and of 0.001, respectively.
[Figure 7] Figure 7 shows the fresh weights of broccoli plants in Example 4. The left graph shows the flower bud weights, and the right graph shows the weights of whole broccoli plants. "a" and "b" above the bars mean that there is no significant difference between bars with identical characters in testing by one-way analysis of variance at a significance level of p < 0.05, and there is significant difference between bars with different characters.
[Figure 8] Figure 8 shows the dry weights of broccoli plants in Example 4. The left graph shows the flower bud weights, and the right graph shows the weights of whole broccoli plants. "a" and "b" above the bars mean that there is no significant difference between bars with identical characters in testing by one-way analysis of variance at a significance level of p < 0.05, and there is significant difference between bars with different characters.

### Description of Embodiments

### [Agricultural Fertilizer]

The agricultural fertilizer of the present embodiment contains a crushed product of a marine purple photosynthetic bacterium and has a nitrogen content of 8.0% by mass or more.

The agricultural fertilizer of the present embodiment is an agricultural fertilizer having a high nitrogen content without depending on any fossil resource.

The nitrogen content of the agricultural fertilizer of the present embodiment is 8.0% by mass or more, preferably 9.0% by mass or more, and more preferably 10% by mass or more.

The upper limit value of the nitrogen content is not particularly limited, and may be, for example, 30% by mass, 20% by mass, or 15% by mass. The nitrogen content may be 8.0% by mass or more and 30% by mass or less, and may be in a range that is within the mentioned range and specified by any lower limit value and any upper limit value selected from those lower and upper limit values.

It is preferable for the agricultural fertilizer of the present embodiment to contain carbon in view of the balance with the nitrogen content, and it is more preferable that the ratio of the carbon content to the value of the nitrogen content described above be within the following range. The ratio of the carbon content to the nitrogen content (C/N) in the mass ratio may be, for example, 1.0 or more and 10.0 or less, preferably 2.0 or more and 8.0 or less, more preferably 2.5 or more and 7.0 or less, and still more preferably 3.0 or more and 7.0 or less. The C/N ratio may be in a range that is within the range of 1.0 or more and 10.0 or less and specified by any lower limit value and any upper limit value selected from those lower and upper limit values.

The nitrogen content and carbon content of an agricultural fertilizer are determined as the total amount of nitrogen and the total amount of carbon in the agricultural fertilizer as measured with a dry combustion method.

The carbon content of the agricultural fertilizer of the present embodiment may be any value that allows the ratio of the carbon content to the nitrogen content (C/N) to fall within the above range without limitation, and is, for example, preferably 30% by mass or more and 80% by mass or less, more preferably 40% by mass or more and 70% by mass or less, and still more preferably 50% by mass or more and 60% by mass or less.

### (Marine Purple Photosynthetic Bacterium)

While purple photosynthetic bacteria can be roughly classified by their habitats into freshwater purple photosynthetic bacteria and marine purple photosynthetic bacteria, a marine purple photosynthetic bacterium, which inhabits marine areas, is used in the present embodiment. Marine purple photosynthetic bacteria are bacteria that can use seawater, nitrogen, carbon dioxide, and light for growing, which are abundant on the earth, and are capable of fixing atmospheric nitrogen with nitrogenase through anoxygenic photosynthesis utilizing carbon dioxide under near-infrared light.

Marine purple photosynthetic bacteria include marine purple sulfur bacteria and marine purple non-sulfur bacteria.

Purple sulfur bacteria are bacteria that perform photosynthesis utilizing near-infrared light to grow in a photoautotrophic manner in the presence of hydrogen, sulfides, and carbon dioxide, and purple non-sulfur bacteria are photosynthetic bacteria that grow in a photoheterotrophic manner in the presence of organic matters and others.

Marine purple sulfur bacteria include bacteria belonging to the genus *Allochromatium* (which may be referred to as *Allochromatium* sp., the same applies hereinafter), bacteria belonging to the genus *Ectothiorhodospira,* bacteria belonging to the genus *Halochromatium,* bacteria belonging to the genus *Halorhodospira,* bacteria belonging to the genus *Marichromatium,* bacteria belonging to the genus *Thiocapsa,* bacteria belonging to the genus *Thiohalocapsa,* and bacteria belonging to the genus *Thiophaeococcus,* and marine purple non-sulfur bacteria include bacteria belonging to the genus *Rhodobaca,* bacteria belonging to the genus *Rhodobacter,* bacteria belonging to the genus *Rhodobium,* bacteria belonging to the genus *Afifella* (*Rhodobium*), bacteria belonging to the genus *Rhodothalassium,* bacteria belonging to the genus *Rhodovulum,* and bacteria belonging to the genus *Roseospira.*

Additional examples of the marine purple photosynthetic bacteria include bacteria disclosed in PLOS ONE | DOI:10.1371/journal.pone.0160981; specifically, purple photosynthetic bacteria that inhabit marine environments among purple photosynthetic bacteria that are disclosed in Table 1 in the article and shown below.

The marine purple photosynthetic bacterium may be any of the marine purple photosynthetic bacteria listed as Organism in Table 1 shown below, specifically, any of *Thiohalocapsa marina, Thiophaeococcus mangrovi, Marichromatium bheemlicum, Afifella marina, Rhodovulum euryhalinum, Rhodovulum imhoffii, Rhodovulum sulfidophilum, Rhodovulum tesquicola, Rhodovulum*

*visakhapatnamense, Roseospira marina,* and *Roseospira goensis;* if there is any change in bacterial names because of alternation of the classification, the marine purple photosynthetic bacterium may be one under the new bacterial nomenclature. The marine purple photosynthetic bacterium may be a bacterium that has a genetic name under that nomenclature and corresponds to any of bacteria belonging to the genus *Thiohalocapsa,* bacteria belonging to the genus *Thiophaeococcus,* bacteria belonging to the genus *Marichromatium,* bacteria belonging to the genus *Afifella,* bacteria belonging to the genus *Rhodovulum,* bacteria belonging to the genus *Roseospira,* and bacteria belonging to the genus *Roseospira.*

**[Table 1]**

| **Sulfur type** | **Resource No.** | **Organism** | **Original marine area** |
|---|---|---|---|
| Sulfur | DSM5653 | *Thiohalocapsa marina* | Mediterranean Sea |
| Sulfur | JCM14889 | *Thiophaeococcus mangrovi* | Orissa, India |
| Sulfur | JCM13911 | *Marichromatium bheemlicum* | Bhimunipatnam, India |
| Non-sulfur | DSM2698 | *Afifella marina* | Kagoshima, Japan |
| Non-sulfur | DSM4868 | *Rhodovulum euryhalinum* | Russia |
| Non-sulfur | JCM13589 | *Rhodovulum imhoffii* | Bhimunipatnam, India |
| Non-sulfur | ATCC35886 | *Rhodovulum sulfidophilum* | Groningen, Netherlands |
| Non-sulfur | ATCC BAA1573 | *Rhodovulum tesquicola* | Soda Lake, Russia |
| Non-sulfur | JCM13531 | *Rhodovulum visakhapatnamense* | Visakhapatnam, India |
| Non-sulfur | ATCC BAA447 | *Roseospira marina* | Arcachon Bay, France |
| Non-sulfur | JCM14191 | *Roseospira goensis* | Goa, India |
| Non-sulfur | ATCC BAA1365 | *Roseospira visakhapatnamensis* | Kakinada, India |
| doi:10.1371/journal.pone.0160981.1001 | | | |

Marine purple sulfure bacteria include *Marichromatium bheemlicum, Thiohalocapsa marina*, and *Thiophaeococcus mangrovi,* and marine purple non-sulfur bacteria include *Afifella pfennigii* (*Rhodobium pfennigii*), *Afifella marina* (*Rhodobium marinum*)*, Rhodovulum euryhalinum, Rhodovulum imhoffii, Rhodovulum sulfidophilum, Rhodovulum tesquicola, Rhodovulum visakhapatnamense, Roseospira goensis,* and *Roseospira marina.*

The marine purple photosynthetic bacterium to be used in the present embodiment may be a marine purple photosynthetic bacterium that is isolated from seawater in Kyoto among the above bacterial species, for example, a bacterium belonging to the genus *Marichromatium.*

Such a marine purple photosynthetic bacterium may be obtained from a depositary institution through a prescribed procedure. The marine purple photosynthetic bacterium may be a mutant of any of the marine purple photosynthetic bacteria shown above. Mutants include ones obtained with a genetic method such as recombination, transduction, and transformation.

In the present embodiment, use of a marine purple photosynthetic bacterium capable of growing under photoheterotrophic conditions or photoautotrophic conditions is preferred, and *R. sulfidophilum* is preferably used.

### (Production of Agricultural Fertilizer)

A crushed product of the marine purple photosynthetic bacterium in the present embodiment is produced with the following method as a mode of implementation.

The marine purple photosynthetic bacterium is cultured while being irradiated with artificial light suitable for photosynthesis, and the cells are then harvested. The harvested marine purple photosynthetic bacterium are crushed or crushed and dried, giving the crushed product of the marine purple photosynthetic bacterium in the present embodiment.

The resulting crushed product of the marine purple photosynthetic bacterium may be used as an agricultural fertilizer.

To obtain the crushed product, one or more treatments of extraction, desalting, granulation, grain size control, and acid/alkali treatment may be performed in addition to culturing, harvesting, crushing, and drying. The marine purple photosynthetic bacterium is preferably contained in the agricultural fertilizer as a powder formed through crushing treatment and subsequent drying treatment.

The agricultural fertilizer of the present embodiment is able to blend well in soil by virtue of the inclusion of the crushed product of the marine purple photosynthetic bacterium.

In the present embodiment, each of the treatments from culturing the marine purple photosynthetic bacterium to giving the crushed product may be appropriately performed through a combination of conventionally known methods, and is preferably in a manner described in the following.

In the present embodiment, an agricultural fertilizer whose nitrogen content is high can be produced through a culturing process for the marine purple photosynthetic bacterium. In the present embodiment, an agricultural fertilizer having a preferable ratio of the carbon content to the nitrogen content can be produced through a culturing process for the marine purple photosynthetic bacterium.

Culturing herein refers to a process of culturing a bacterium under specific conditions to increase the number of bacterial cells and allow nutrients such as protein (in particular, carbon components and nitrogen components) to be accumulated in cells of the bacterium.

Methods known as large-scale culture methods may be employed as a culture method for use in the present embodiment, and examples include continuous culture methods and batch culture methods.

Culture of a starter and culture of the marine purple photosynthetic bacterium to obtain a crushed product of the marine purple photosynthetic bacterium may be appropriately performed without limitation, and culturing the marine purple photosynthetic bacterium under specific conditions for growing it is preferred in the culturing process in the present embodiment.

Culture may be performed under irradiation with near-infrared light which the marine purple photosynthetic bacterium uses to grow in a photoautotrophic manner, and far-red light may be used.

The far-red light may be light the peak wavelength of which lies in a wavelength region of 700 nm to 860 nm.

For irradiating with far-red light, an irradiation method that is used in conventional culture of a marine purple photosynthetic bacterium may be used without limitation.

For culture time, culture may be performed for a period of time enough to allow the marine purple photosynthetic bacterium to accumulate biological substances such as protein, and a culture temperature may be appropriately determined depending on the optimum culture temperature of the marine purple photosynthetic bacterium. The culture temperature may be, for example, 20 to 40°C.

For culture time, for example, the following culture may be performed: culture is performed until the intermediate logarithmic growth phase is reached, medium exchange or the like is then performed, culture is subsequently performed, and culture is performed until the stationary phase is reached.

For the growth or the like of the marine purple photosynthetic bacterium, measurements of optical cell density based on absorbance at 660 nm (OD₆₆₀) may be used as the index.

While culture may be performed under an appropriate atmosphere, culture under conditions with nitrogen allows fixation of atmospheric nitrogen, and a nitrogen-rich agricultural fertilizer can be provided even without addition of a nitrogen source into the medium. The medium may be bubbled with nitrogen gas to increase the nitrogen concentration in the medium.

The medium for culture may be any medium that allows the marine purple photosynthetic bacterium to be cultured without limitation, and a conventionally known growth medium may be used. Natural seawater may be used as the medium for culture without limitation, and the medium may be a seawater-based medium with use of natural seawater.

The growth medium may contain an organic carbon source, and may contain an inorganic carbon source. If containing an inorganic carbon source, the medium may lack an organic carbon source.

An inorganic carbon source is preferably used in the present embodiment, and in some cases carbon fixation may be promoted through culturing in a medium without any organic carbon source.

The organic carbon source may be, for example, any of glucose, fructose, sucrose, and syrup containing them, carbohydrates including starch and starch hydrolysates and the like, organic acids including acetic acid and propionic acid, and alcohols including ethanol and propanol.

The inorganic carbon source may be, for example, carbon dioxide, carbonate ions, bicarbonate ions, or carbon monoxide. Carbonate ions or bicarbonate ions may be added as a metal salt into the medium. Carbon dioxide or carbon monoxide may be added by means of bubbling together with or separately from nitrogen gas.

If the culture atmosphere contains nitrogen, the growth medium does not need to contain a nitrogen source. The growth medium may contain a nitrogen source. Applicable as the nitrogen source are, for example, ammonia, ammonium salts of an inorganic acid or an organic acid such as ammonium chloride, ammonium sulfate, ammonium acetate, and ammonium phosphate, other nitrogen-containing compounds, peptone, meat extracts, yeast extracts, corn steep liquors, casein hydrolysates, soybean meal and soybean meal hydrolysates, and fermentative bacterial cells and digested products thereof.

For each of the organic carbon source, inorganic carbon source, and nitrogen source, one substance or two or more substances may be used.

The growth medium may further contain an inorganic salt. Examples of the inorganic salt include monobasic potassium phosphate, dibasic potassium phosphate, magnesium phosphate, magnesium sulfate, sodium chloride, sodium thiosulfate, ferrous sulfate, manganese sulfate, copper sulfate, and calcium carbonate.

An example of preferred media is Marine Broth medium described in Example. An oligotrophic medium may be used. Examples of the oligotrophic medium include M6 medium and a medium obtainable by adding appropriate additives to natural seawater. Examples of additives that may be added to natural seawater include lignin-containing waste liquid (what is called lignin waste liquid) discarded in the papermaking process for Japanese paper, sodium thiosulfate, and the nitrogen sources shown above. Particularly preferable nitrogen sources to be added to natural seawater are peptone and yeast extract. Such additives may be each independently added, for example, at a concentration of 0.01% or more and 5.0% or less, preferably of 0.01% or more and 3.0% or less.

The composition of M6 medium is, for example, sodium malate: 5 g; KH₂PO₄: 0.75 g; K₂HPO₄: 0.78 g; CaCl₂·2H₂O: 0.029 g; MgSO₄·7H₂O: 0.247 g; (NH₄)₂SO₄: 1 g; FeSO₄·7b₂O: 0.011 g; vitamin solution: 10 mL; and trace element solution: 10 µL. Here, the composition of the vitamin solution per 100 mL is nicotinic acid: 0.1 g, thiamine: 0.1 g, biotin: 0.005 g, para-aminobenzoic acid: 0.05 g, vitamin B₁₂: 0.001 g, vitamin B₅: 0.05 g, pyridoxine hydrochloride: 0.05 g, EDTA·3Na: 0.2 g, folic acid: 0.05 g, ZnCl₂·5H₂O: 70 µg, MnCl₂·4H₂O: 100 µg, H₃BO₃: 60 µg, CoCl₂·6H₂O: 200 µg, CuCl₂·2H₂O: 20 µg, NiCl₂·6H₂O: 20 µg, and Na₂MoO₄·H₂O: 40 µg. The composition of the trace elements per 1 L is MnSO₄·4H₂O: 11.16 g, ZnSO₄·7H₂O: 2.88 g, Co(NO₃)₂·6H₂O: 2.92 g, CuSO₄·5H₂O: 2.52 g, Na₂MoO₄·2H₂O: 2.42 g, H₃BO₃: 3.10 g, and EDTA·3Na: 41.20 g.

The marine purple photosynthetic bacterium is cultured while being irradiated with artificial light suitable for photosynthesis, and the cells are then preferably collected and washed, and then harvested. In harvesting, washing may be followed by collection; rather, collection may be followed by washing. In harvesting, cells may be harvested from one or more culture tanks. Collection and washing may be each performed multiple times.

Collection of cells of the marine purple photosynthetic bacterium may be performed with a conventionally known method for collecting cells of a bacterium from culture solution.

Washing of cells of the marine purple photosynthetic bacterium may be performed once through suspending collected bacterial cells in a desired solution, or performed with desalting by a conventionally known method such as ultrafiltration.

The number of times of collection and/or washing for harvesting cells of the marine purple photosynthetic bacterium is not particularly limited, and may be one or more.

Desalting to remove salts present in the culture solution or washing solution or acid/alkali treatment for the purpose of adjusting the pH of the culture solution or washing solution may be performed with a conventionally known method.

The marine purple photosynthetic bacterium is contained in the agricultural fertilizer as a crushed product formed through crushing treatment.

The crushing may be, for example, mechanical crushing with a mortar or the like, crushing with a sonicator, crushing with a homogenizer, crushing with a bead mill homogenizer, enzymatic crushing, crushing by freeze-thawing, crushing with an organic solvent (preferably, with acetone), or a combination of any of them. The crushing treatment may be performed with a method of homogenization treatment, for example, using a high-pressure homogenizer at a pressure of 800 to 1500 bar in one or more cycles (e.g., in 3 to 10 cycles, preferably in five cycles). The crushing treatment may be performed with a method of homogenization treatment, for example, using an ultrasonic homogenizer in one or more cycles. The crushing treatment may be performed, for example, with a method in which a culture of the bacterium is suspended in acetone, then dried under the atmosphere, and thereafter further mechanically crushed with a mortar or the like.

In manufacturing the agricultural fertilizer, drying treatment may be performed, and the drying treatment may be performed, for example, with a forced- air drying method to blow warm air or cool air with a blower or the like, a windless drying method to evaporate moisture through heating, spray drying in which a slurry is suspended in an appropriate buffer and the suspension is then sprayed into gas for rapid drying, a freeze-drying method, a vacuum drying method to deaerate a sealed container with a vacuum pump or the like, a natural drying method to leave in the open air (including sun-drying), or a combination of any of them. The drying treatment may be, for example, by means of freeze-drying or a natural drying method.

The agricultural fertilizer of the present embodiment is preferably provided as a solid fertilizer.

The crushed product of the marine purple photosynthetic bacterium may be used alone for the agricultural fertilizer, or appropriately treated together with other components, as necessary, and used for the agricultural fertilizer. The agricultural fertilizer of the present embodiment may be mixed with a conventionally known agricultural fertilizer for use.

### [Method for Growing Agricultural Crop]

A mode of the present embodiment is a method for growing an agricultural crop by using the agricultural fertilizer. Examples of the target agricultural crop in this method include those as shown below, and the agricultural fertilizer may be in any of the modes described above.

The present method includes growing an agricultural crop in soil, medium, or culture solution or the like containing the agricultural fertilizer. The agricultural fertilizer of the present embodiment may be added once, or twice or more times at appropriate intervals to the soil, medium, or culture solution or the like.

The agricultural fertilizer of the present embodiment may have slow-release properties, and may exert the slow fertilizer effect to an agricultural crop by one addition. The agricultural fertilizer of the present embodiment may be used for the purpose of soil improvement by slowly releasing nitrogen over a period longer than the cropping season.

The agricultural fertilizer of the present embodiment may keep acting as a fertilizer to continuously supply nitrogen components to soil at least for 15 days or more, preferably for 20 days or more, more preferably for 30 days or more, still more preferably for 35 days or more. The duration of the action of the agricultural fertilizer of the present embodiment may be, for example, 5 years or less, 3 years or less, 1 year or less, 10 months or less, 8 months or less, 6 months or less, 5 months or less, 4 months or less, or 3 months or less.

In the method for growing an agricultural crop, adjustment of the amount of usage of the agricultural fertilizer of the present embodiment allows nutrients, in particular, nitrogen to be supplied in necessary and sufficient amounts for the agricultural crop to grow. In the case of adding to soil, for example, the agricultural fertilizer of the present embodiment may be blended to give a nitrogen level of 0.01 g/L or more and 1.0 g/L or less or 0.03 g/L or more and 0.50 g/L or less.

The target to grow with the agricultural fertilizer of the present embodiment is not particularly limited, and, for example, selected from angiosperms and gymnosperms. Examples of the agricultural crop include, but are not limited to, grains, vegetables, fruits, flowers, beans, and tea, and leafy vegetables such as Japanese mustard spinach, spinach, parsley, and bok choy are preferred.

Being an agricultural fertilizer having a high nitrogen content, the agricultural fertilizer of the present embodiment is preferably applied to a plant that highly requires nitrogen or a plant in a stage when the plant highly requires nitrogen. It is preferred to grow an agricultural crop that requires much nitrogen for the growth such as eggplants, tea, and ebi-imo (shrimp-shaped taro).

Examples of the target plant include, but are not limited to, plants belonging to the family Asteraceae such as chrysanthemums and gerberas, plants belonging to the family Solanaceae such as potatoes, tomatoes, and eggplants, plants belonging to the family Brassicaceae such as Japanese mustard spinach, broccoli, rape, and rapeseed, plants belonging to the family Poaceae such as rice, maize, wheat, sugarcane, and barley, plants belonging to the family Leguminosae such as soybean, plants belonging to the family Convolvulaceae such as morning glory and sweet potatoes, plants belonging to the family Salicaceae such as poplars, plants belonging to the family Euphorbiaceae such as castor-oil plants, cassava, and *Jatropha curcas,* plants belonging to the family Rutaceae such as oranges and lemons, plants belonging to the family Rosaceae such as cherry trees and roses, plants belonging to the family Orchidaceae such as phalaenopsis, plants belonging to the family Gentianaceae such as Texas bluebell, plants belonging to the family Primulaceae such as cyclamen, plants belonging to the family Violaceae such as pansies, plants belonging to the family Liliaceae such as lilies, plants belonging to the family Amaranthaceae such as sugar beets, plants belonging to the family Vitaceae such as grapes, plants belonging to the family Cupressaceae such as Japanese cedars and Hinoki cypresses, plants belonging to the family Oleaceae such as olives and fragrant olives, and plants belonging to the family Pinaceae such as Japanese red pines.

The target to grow may be, for example, a leafy vegetable, and may be a plant belonging to the family Brassicaceae, in particular, Japanese mustard spinach or broccoli.

The agricultural fertilizer of the present embodiment may be applied to the whole or a part of a plant at any of the growth stages including the pre-germination and post-germination stages of the plant (e.g., the whole or a part of a seed, a seedling, or a mature plant).

The agricultural fertilizer of the present embodiment may promote the growth of a target agricultural crop (plant), and may enhance the stress resistance such as thermal resistance, drought resistance, and salt tolerance. The agricultural fertilizer of the present embodiment may swell and/or increase at least a part of an agricultural crop (plant); specifically, the agricultural fertilizer of the present embodiment may achieve any of: enlarging at least a part of an agricultural crop; increasing the number of at least one of the organs of an agricultural crop; shortening the time for at least a part of an agricultural crop to reach a specific size as compared with the case without giving the agricultural fertilizer; and shortening the time for the number of at least one of the organs of an agricultural crop to reach a specific number as compared with the case without giving the agricultural fertilizer. The agricultural fertilizer may have a growth-promoting effect such as elongation of stems and leaves or roots, increase in the number of leaves, promotion of flowering or fruiting, increase in the number of flowers or fruits, increase in plant body weights or crop yields, greening, and promotion of tillering.

### Example

The following specifically describes the present invention with an example and comparative examples. The present invention is by no means limited to the following example.

### [Example 1]

### (Bacterial Culture)

The marine purple photosynthetic bacterium *R*. *sulfidophilum* was obtained from the American Type Culture Collection (ATCC).

For culturing the marine purple photosynthetic bacterium, Marine Broth 2216 medium (manufactured by Sigma-Aldrich Co. LLC) was used. The composition of the Marine Broth medium per 1 L was as follows: NH₄NO₃: 1.6 mg, H₃BO₃: 22.0 mg, CaCl₂: 1.8 g, Na₂HPO₄: 8.0 mg, iron(III) citrate: 0.1 g, MgCl₂: 5.9 g, MgSO₄: 3.24 g, peptone: 5.0 g, KBr: 0.08 g, KCl: 0.55 g, NaHCO₃: 0.16 g, NaCl: 19.45 g, NaF: 2.4 mg, sodium silicate: 4.0 mg, SrCl₂: 34.0 mg, and yeast extract: 1.0 g.

For culture and preparation of a starter culture, an agar culture colony of the bacterium was cultured in 50 mL of Marine Broth 2216 medium in a sterilized screwcap tube (atmospheric conditions).

Until the optical cell density based on optical density at 660 nm (OD₆₆₀) reached 1 to 1.5 (intermediate logarithmic growth phase), culture was maintained under static conditions at 30°C under a far-red light LED (730 nm, 20 Wm⁻², manufactured by CCS Inc.). Subsequently, the culture was transferred into 0.5 to 1 L of Marine Broth medium, and maintained under the same conditions with stirring with a magnetic stirrer at a stirring speed of 200 rpm until the cells reached the intermediate logarithmic growth phase. Thereafter, the culture was transferred into 10 L of Marine Broth medium, and cultured under the same conditions with stirring at a stirring speed of 450 rpm until the cells reached the stationary phase (optical cell density at OD₆₆₀: 1.8 to 2.0).

### (Production of Agricultural Fertilizer)

With the bacterium cultured as described above, an agricultural fertilizer was produced. After repeating washing and harvesting multiple times, the purified cells of the bacterium were homogenized five times with the high-pressure homogenizer Panda Plus1000 at a pressure of 1000 bar. Thereafter, the resultant was freeze-dried to give an agricultural fertilizer (referred to as "PhotoB" in Example 1 shown later).

### (Component Analysis)

Component analysis of the agricultural fertilizer (PhotoB) thus produced was carried out as follows. The result is shown in Table 2 shown below.
· pH (H₂O): 1:10 water shaking extraction and glass electrode method
· Total nitrogen: dry combustion method
· Total phosphate: nitric acid decomposition (microwave decomposition) and absorptiometry (ammonium vanadomolybdate method)
· Total potassium: nitric acid decomposition (microwave decomposition) and atomic absorptiometry · Total carbon: dry combustion method

**[Table 2]**

| Analysis item | Analytical value | Unit |
|---|---|---|
| pH (H₂O) | 6.7 | pH |
| Total nitrogen (N) | 11.2 | % |
| Total phosphate (P₂O₅) | 2.31 | % |
| Total potassium (K₂O) | 0.58 | % |
| Total carbon (C) | 52.9 | % |

Thus, it was confirmed that the marine purple photosynthetic bacterium can be continuously cultured at a 10-L scale. In addition, the fertilizer derived from the cultured marine purple photosynthetic bacterium was found to contain nitrogen components in amounts sufficient for plants to grow.

As demonstrated in Table 2, PhotoB, the agricultural fertilizer derived from the marine purple photosynthetic bacterium, was found to have relatively low phosphorus and potassium contents, probably being able to serve as an organic fertilizer to supply nitrogen in a preferable manner to gardens containing much available phosphate and thus containing excessive phosphate and soils containing much exchangeable potassium and thus containing potassium too much.

### (Growth Test)

Soil for farming in a farm belonging to the Graduate School of Agriculture, Kyoto University was air-dried in the shade and passed through a 2-mm sieve, and mixed with an inorganic fertilizer or PhotoB at a ratio shown below, and the resultant was used as culture soil.

In control plots, soil mixed with a fast-acting inorganic fertilizer (N:P:K = 8:8:8, Akagi Engei) at a rate of 1 g/L (control reference plot (C1)) or 2 g/L (control 2-fold plot (C2)) was used. Soil mixed with calcium superphosphate (soluble phosphate: 17.5%, Akagi Engei) and potassium chloride (NACALAI TESQUE, INC.) to give the same amounts of phosphorus and potassium as in control reference plot C1 was used as a non-nitrogen-fertilized plot (Neg).

In experimental plots (Example), PhotoB (N:P:K = 11.2:2.31:0.58) was mixed to give a total N content 0.5, 1, 2, or 4 times as much as that in control reference plot C1 ("PhotoB (0.5N)", "PhotoB (1N)", "PhotoB (2N)", "PhotoB (4N)", respectively). For PhotoB (1N), PhotoB was mixed at 0.71 g/L.

The amounts of nitrogen, phosphorus, and potassium added to the plots are summarized in Table 3 in the following.

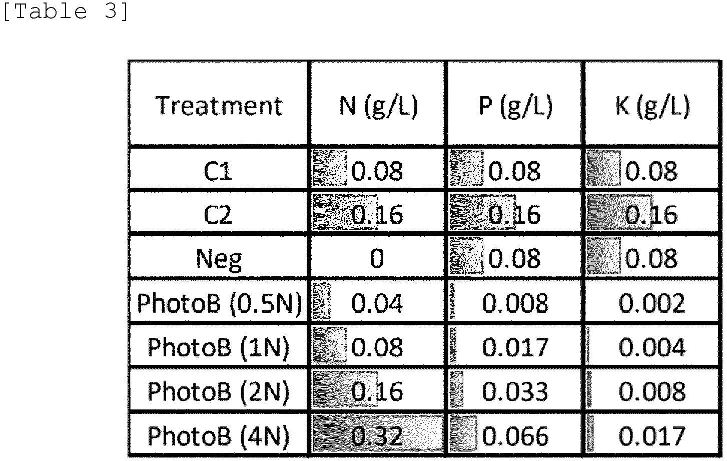

Seeds of Japanese mustard spinach (Natsu Raku-Ten, TAKII & CO., LTD.) were seeded for cultivation in each of the culture soils under the conditions described above, and the growths were observed.

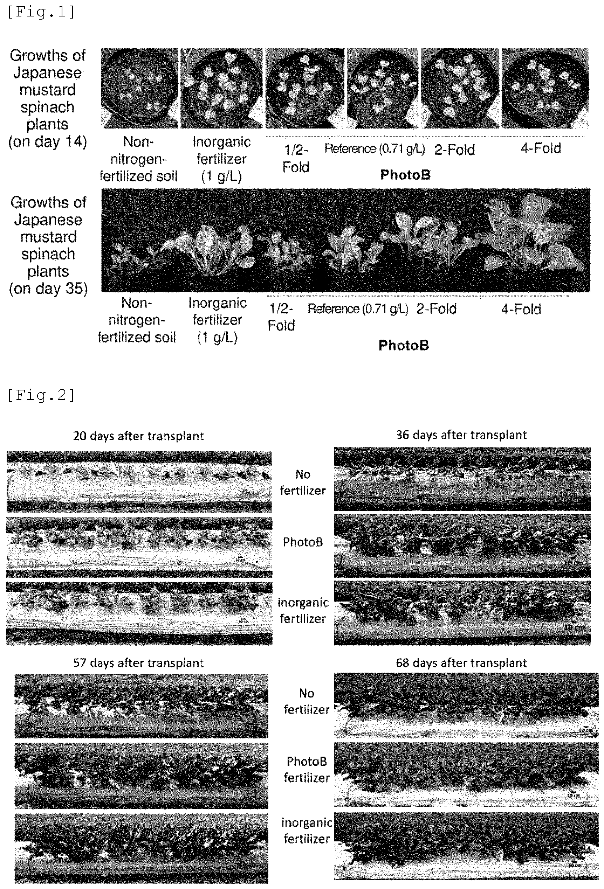

Figure 1 shows observation photographs of Japanese mustard spinach plants cultivated for 14 days and those for 35 days under the respective conditions. Comparison of the growth of Japanese mustard spinach plants 14 days after sowing found that the Japanese mustard spinach plants to which the fertilizer derived from the marine purple photosynthetic bacterium had been given exhibited superior growth to those in the non-nitrogen-fertilized soil. In addition, the growth was found to be almost the same as those in the soil with the inorganic blend fertilizer applied. On day 35, the Japanese mustard spinach plants to which the fertilizer derived from the marine purple photosynthetic bacterium had been given exhibited sound growth comparable to those in the soil with the inorganic blend fertilizer applied, and the growth increased according to the amount of the fertilizer. Thus, the fertilizer derived from the marine purple photosynthetic bacterium was revealed to be not only capable of relatively short-term nitrogen supply but also able to gradually supply nitrogen components to agricultural crops. This suggests that the fertilizer derived from the marine purple photosynthetic bacterium has slow-release properties.

### [Example 2]

Examination of the culture conditions for the marine purple photosynthetic bacterium was performed. As in Example 1, the marine purple photosynthetic bacterium *R*. *sulfidophilum* was obtained from ATCC.

The marine purple photosynthetic bacterium was cultured in eight batches as follows. Each batch was a 500-L pond culture with 30 L of the marine purple photosynthetic bacterium precultured in Marine Broth medium. Culture conditions for the batches were as shown in a table given below. In each batch, an acetic acid solution containing chitosan, as shown in the table given below, was added as a flocculant on day 5 of culture. The table given below shows yields of cultures in the batches.

Culture was performed with irradiation of light by using: a halogen lamp placed above in batches 1 to 3; a halogen lamp placed above and a laterally placed halogen lamp, two halogen lamps in total, in batch 4; and a halogen lamp placed above and a laterally placed halogen lamp, two halogen lamps in total, and laterally placed three LED tube lights and three LED panels in batches 5 to 8.

**[Table 4]**

| Batch | 1 | 2 | 3 | 4 |
|---|---|---|---|---|
| Culture solution (% by mass) | | NSW | NSW | NSW |
| | NSW | 1% LW | 1% LW | |
| | 1% LW | 0.04% STS | 0.04% YE | 0.04% YE |
| | 0.04% STS | 0.01% YE | 0.05% Peptone | 0.2% Peptone |
| | | 0.025% Peptone | | |
| Chitosan solution | 5 mg/L Chitosan 100 (1% acetic acid) | 10 mg/L Chitosan 100 (8.75% acetic acid) | 10 mg/L Chitosan 100 (9% acetic acid) | 10 mg/L Chitosan 100 (1% acetic acid) or 10 mg/L chitosan 10 (1% acetic acid) |
| Yield of culture (g/L) | 0.88 | 1.06 | 1. 09 | 1.13 |

**[Table 5]**

| Batch | 5 | 6 | 7 | 8 |
|---|---|---|---|---|
| Culture solution (% by mass) | NSW | NSW | NSW | NSW |
| | | 0.04% STS | 0.04% STS | 0.04% STS |
| | 0.1% YE | 0.1% YE | 0.05% YE | 0.05% YE |
| | 0.5% Peptone | 0.1% Peptone | 0.03% Peptone | 0.05% Peptone |
| Chitosan solution | 10 mg/L Chitosan 10 (1% acetic acid) | 10 mg/L Chitosan 100 (1% acetic acid) | 10 mg/L Chitosan 100 (1% acetic acid) | 10 mg/L Chitosan 100 (1% acetic acid) |
| Yield of culture (g/L) | 2.03 | 2.00 | 1.75 | 1.40 |

The meanings of abbreviations in the tables are as follows.
NSW: Natural sea water (obtained from Maizuru city, Kyoto prefecture, Japan)
LW: Lignin waste liquid (obtained from paper mill for Japanese paper)
STS: Sodium thiosulfate
YE: Yeast extract

Cells of the bacterium cultured as described above were applied to a centrifuge (HIMAC) at 8000 rpm for 5 minutes for separation. Thus, the marine purple photosynthetic bacterium was confirmed to allow culture at a 500-L scale.

### [Example 3]

Examination was performed on crushing methods for the marine purple photosynthetic bacterium. Crushed products of the bacterium were obtained through three protocols shown below. Elemental analysis of the crushed products confirmed the crushed products given through each of the protocols to have a nitrogen content and carbon content comparable to those in Example 1.

Protocol 1: After culturing in Marine Broth medium, the resultant was subjected to homogenization treatment with an ultrasonic homogenizer, and then freeze-dried.

Protocol 2: After culturing in Marine Broth medium, cells of the bacterium were resuspended in acetone at 2 g/mL and crushed, dried in the atmosphere at 24°C, and further triturated with a mortar. Thereafter, the crushed product was passed through a 2-mm sieve.

Protocol 3: After culturing in Marine Broth medium supplemented with chitosan, cells of the bacterium were resuspended in acetone at 2 g/mL and crushed, dried in the atmosphere at 24°C, and further triturated with a mortar. Thereafter, the crushed product was passed through a 2-mm sieve.

### [Example 4]

### (Production of Agricultural Fertilizer)

The marine purple photosynthetic bacterium was cultured in Marine Broth medium at a 10-L scale as in Example 1, giving 664 g of biomass. The cultures of batches 1 to 5 in Example 2 were combined to give 1.76 kg of biomass. Those cultures were mixed together, and a 875-g portion thereof was crushed to give an agricultural fertilizer (referred to as "PhotoB" in the following in Example 4). In Example 4, a part of the culture was crushed with a method in which homogenization treatment with an ultrasonic homogenizer was followed by freeze-drying, and the rest of the culture was crushed with a method in which cells of the bacterium were resuspended in acetone and crushed, dried in the atmosphere, and then further triturated with a mortar. These crushed products were mixed together, and the resultant was used as an agricultural fertilizer.

### (Growth Test)

Growth test was carried out for broccoli plants (Green Canon, SAKATA SEED CORPORATION) as follows.

The growing schedule was as follows.
Seeding: August 2, 2022
Transplanting to pots: August 30, 2022
Field transplant: September 15, 2022
Harvesting: December 5, 2022

Cultivation was performed with cell trays from seeding to potting-up, and with 9-cm pots from potting-up to field transplant. Cultivation was performed with commercially available cultivation soil ("Iku-byo Baido" manufactured by TAKII & CO., LTD.) from seeding to field transplant.

After field transplant, 10 broccoli plants were planted in each of three treatment plots shown in Table 6 given below. Konohana UF is "Konohana (R)-jirushi UF Kasei", a slow-release fertilizer manufactured by Sumitomo Chemical Co., Ltd. with N-P-K contents of 16-10-14. Before ridging, BM YORIN was added to all the treatment plots at 100 kg/10 a.

Thereafter, additional fertilizers were given to all the treatment plots according to the following schedule.
October 11, 2022: RIN-SHOAN-KARI S604 (N-P-K contents: 16-10-14) manufactured by JCAM AGRI. CO., LTD., 15 kg/ 10 a
October 20, 2022: Sumitomo Liquid Fertilizer No. 2 (N-P-K contents: 10-5-8) manufactured by Sumitomo Chemical Co., Ltd., applying 100 L of 100-fold-diluted product to three treatment plots (100 L/30 individuals)

**[Table 6]**

| Treatment plot | Type of base fertilizer | Amount of base fertilizer |
|---|---|---|
| 1 (Comparative Example) | None | - |
| 2 (Reference Example) | Konohana UF | 80 kg/10 a |
| 3 (Example) | PhotoB | Adjusted to give a total nitrogen content of 12.8 kg/10 a, which was the same as that in treatment plot 2 (Reference Example) (total amount to 6 m² of cultivation area: 700 g) |

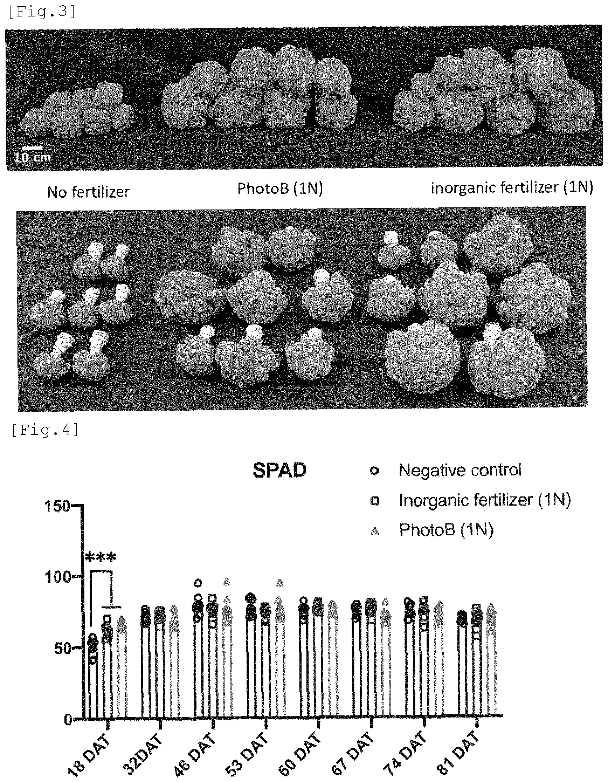

Figure 2 shows observation photographs of the broccoli plants cultivated in the treatment plots. Figure 3 shows observation photographs of broccoli plants harvested on day 81 after field transplant. In Figures 2 and 3, "inorganic fertilizer" indicates the treatment plot with Konohana UF.

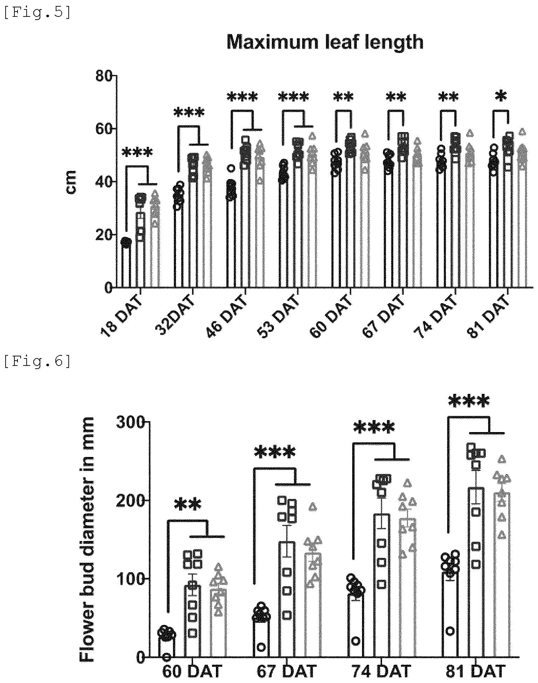

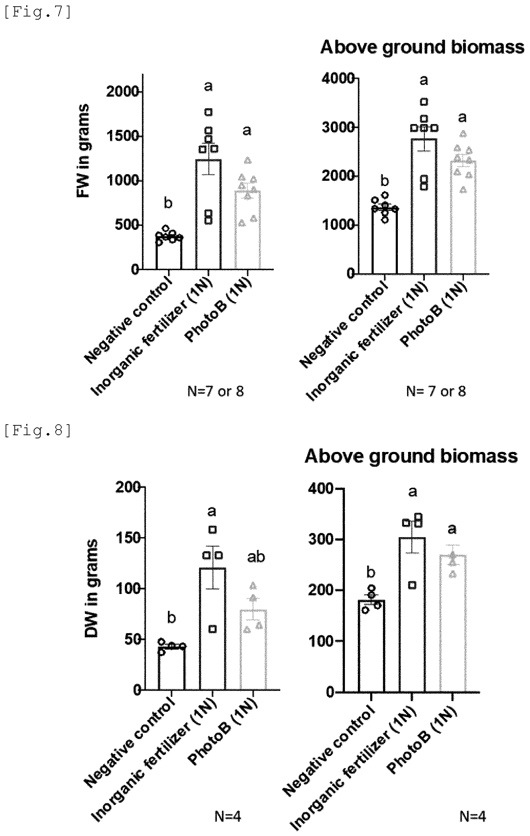

To compare the growth states of the broccoli plants cultivated in the treatment plots, SPAD values, maximum leaf lengths, and maximum flower bud diameters were measured every several days after transplant. SPAD values were measured with a SPAD meter. After harvesting on day 81 after field transplant, the fresh weights (FW) and dry weights (DW) were measured. The dry weights were weights measured after drying at 80°C for 14 days or more. The results are shown in Figures 4 to 8. The legends in Figure 4 also apply to Figures 5 to 8, and the legend "Inorganic fertilizer" indicates the broccoli plants cultivated in the treatment plot with Konohana UF. In Figures 4 to 8, DAT means the number of days after field transplant (Day After Transplant).

The results suggested that the fertilizer derived from the marine purple photosynthetic bacterium, a zero-carbon-based fertilizer produced with light, atmospheric nitrogen, and carbon dioxide, serves as an alternative to nitrogen raw materials of inorganic blend fertilizers.

### Industrial Applicability

The agricultural fertilizer of the present invention can contribute to spread and development of the next-generation agriculture as a new option of sustainable, organic fertilizers alternative to chemical fertilizers. In addition, the agricultural fertilizer of the present invention is applicable to greening of desertified areas and promotion of dry farming.

## Claims

1. An agricultural fertilizer comprising a crushed product of a marine purple photosynthetic bacterium and having a nitrogen content of 8.0% by mass or more.

2. The agricultural fertilizer according to claim 1, wherein a ratio of a carbon content to the nitrogen content (C/N) is 3.0 or more and 7.0 or less.

3. The agricultural fertilizer according to claim 1 or 2, wherein the agricultural fertilizer is a solid fertilizer.

4. The agricultural fertilizer according to claim 1 or 2, wherein the agricultural fertilizer has slow-release properties and thereby continuously acts for 30 days or more.
